# EUROPEAN PATENT SPECIFICATION

(11) **EP 4 388 989 B1**
(45) Date of publication and mention of the grant of the patent: **11.03.2026**
(21) Application number: 23202252.5
(22) Date of filing: 06.10.2023
(51) Int. Cl.: A61B 5/0205, A61B 5/08, A61B 5/18, A61B 5/00

(54) **SYSTEMS FOR DETECTING POTENTIAL PHYSIOLOGICAL EPISODES BASED ON SENSED PILOT RESPIRATION DATA**
SYSTEME ZUR ERKENNUNG POTENZIELLER PHYSIOLOGISCHER EPISODEN AUF BASIS ERFASSTER PILOTATMUNGSDATEN
SYSTÈMES DE DÉTECTION D'ÉPISODES PHYSIOLOGIQUES POTENTIELS SUR LA BASE DE DONNÉES DE RESPIRATION PILOTE DÉTECTÉES

(30) Priority: 22.12.2022 US 202218145544
(43) Date of publication of application: 26.06.2024
(73) Proprietor: Honeywell International Inc., Charlotte, NC 28202 (US)
(72) Inventor: LUBOLD, Nichola, Charlotte, 28202 (US)
(74) Representative: Ingrassia, Fisher & Lorenz UK Ltd.

(56) References cited:
- US-A1- 2005 202 375
- US-A1- 2021 007 647
- US-B1- 10 561 863

## Description

### TECHNICAL FIELD

The present invention generally relates to aircraft operations and more particularly relates to systems for detecting potential physiological episodes based on sensed pilot respiration data.

### BACKGROUND

Physiological episodes represent one of the top safety priorities for the U.S. Navy and the U. S. Airforce. Physiological episodes typically occur when a pilot experiences a loss in performance due to physiological issues that may occur during a flight. Examples of physiological issues include, but are not limited to, hypoxia (resulting from a drop in oxygen levels), hypocapnia (resulting from a drop in blood carbon dioxide levels), hypothermia (resulting from a drop in body temperature), or atelectasis (resulting from experiencing significant g-forces). Degraded pilot performance may pose a safety issue that could potentially lead to an adverse aircraft event. Exemplary pilot monitoring systems are disclosed by US 2021/007647 A1 and US 2005/202375 A1.

Hence there is a need for systems for detecting an onset of potential physiological episodes based on sensed pilot respiration data so that appropriate action can be implemented by the pilot or by automation.

### BRIEF SUMMARY

This summary is provided to describe select concepts in a simplified form that are further described in the Detailed Description. The invention is defined by the appended claims.

An exemplary embodiment of a system includes a processor and a memory. The memory includes instructions that upon execution by the processor, cause the processor to: receive raw physiological data from at least one sensor of a biosensing garment of a pilot, the raw physiological data including raw respiration data; extract individual breaths from the raw physiological data, each individual breath having an associated breath pattern and an association with a breath timeline; receive aircraft environment data associated with an aircraft from at least one aircraft environment sensor, the aircraft environment data being associated with an aircraft environment timeline; align the individual breaths with the aircraft environment data in accordance with the breath timeline and the aircraft environment timeline; classify each individual breath as one of a plurality of different breath types based on the associated breath pattern; determine whether the breath types associated with the individual breaths are associated with a physiological episode (PE) profile based at least in part on the aircraft environment data in accordance with the aligned breath and aircraft timelines; and issue a PE alert based on the determination.

Furthermore, other desirable features and characteristics of the system for detecting potential physiological episodes based on sensed pilot respiration data will become apparent from the subsequent detailed description and the appended claims, taken in conjunction with the accompanying drawings and the preceding background.

### BRIEF DESCRIPTION OF THE DRAWINGS

The present disclosure will hereinafter be described in conjunction with the following drawing figures, wherein like numerals denote like elements, and wherein:
FIG. 1 is a block diagram representation of a system for implementing detection of potential physiological episodes based on sensed pilot respiration data;
FIG. 2 is a block diagram representation of an aircraft including an embodiment of a physiological episode detection system; and
FIG. 3 is a flowchart representation of an exemplary embodiment of a method (not part of the invention) of detecting potential physiological episodes based on sensed physiological data of a pilot and sensed aircraft environment data; and
FIG. 4 is a flowchart representation of an exemplary embodiment of a method (not part of the invention) of detecting potential physiological episodes based on sensed pilot respiration data.

### DETAILED DESCRIPTION

The following detailed description is merely exemplary in nature. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

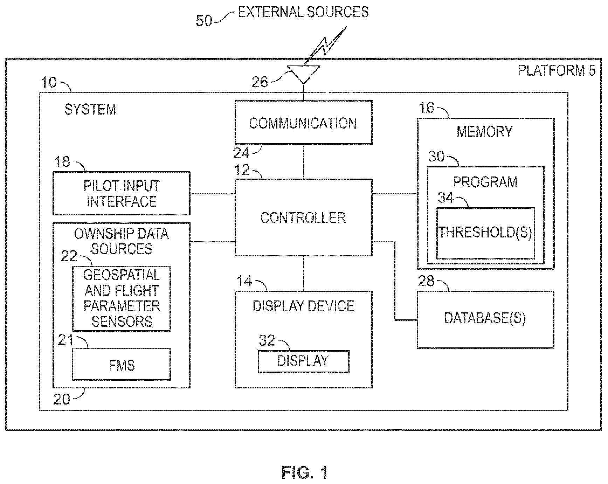

FIG. 1 is a block diagram representation of a system 10 for implementing detection of potential physiological episodes (PE) based on sensed pilot respiration data (shortened herein to "system" 10), as illustrated in accordance with an exemplary and non-limiting embodiment of the present disclosure. The system 10 may be utilized onboard a mobile platform 5, as described herein. In various embodiments, the mobile platform is an aircraft, which carries or is equipped with the system 10. As schematically depicted in FIG. 1, the system 10 includes the following components or subsystems, each of which may assume the form of a single device or multiple interconnected devices: a controller circuit 12 operationally coupled to: at least one display device 14; computer-readable storage media or memory 16; an optional input interface 18, and ownship data sources 20 including, for example, a flight management system (FMS) 21 and an array of flight system state and geospatial sensors 22.

In various embodiments, the system 10 may be separate from or integrated within: the flight management system (FMS) 21 and/or a flight control system (FCS). Although schematically illustrated in FIG. 1 as a single unit, the individual elements and components of the system 10 can be implemented in a distributed manner utilizing any practical number of physically distinct and operatively interconnected pieces of hardware or equipment. When the system 10 is utilized as described herein, the various components of the system 10 will typically all be located onboard the mobile platform 5.

The term "controller circuit" (and its simplification, "controller"), broadly encompasses those components utilized to carry-out or otherwise support the processing functionalities of the system 10. Accordingly, the controller circuit 12 can encompass or may be associated with a programmable logic array, application specific integrated circuit or other similar firmware, as well as any number of individual processors, flight control computers, navigational equipment pieces, computer-readable memories (including or in addition to the memory 16), power supplies, storage devices, interface cards, and other standardized components. In various embodiments, the controller circuit 12 embodies one or more processors operationally coupled to data storage having stored therein at least one firmware or software program (generally, computer-readable instructions that embody an algorithm) for carrying-out the various process tasks, calculations, and control/display functions described herein. During operation, the controller circuit 12 may be programmed with and execute the at least one firmware or software program, for example, a program 30, that embodies an algorithm described herein for detecting of potential physiological episodes (PE) based on sensed pilot respiration data on a mobile platform 5, where the mobile platform 5 is an aircraft, and to accordingly perform the various process steps, tasks, calculations, and control/display functions described herein.

The controller circuit 12 may exchange data, including real-time wireless data, with one or more external sources 50 to support operation of the system 10 in embodiments. In this case, bidirectional wireless data exchange may occur over a communications network, such as a public or private network implemented in accordance with Transmission Control Protocol/Internet Protocol architectures or other conventional protocol standards. Encryption and mutual authentication techniques may be applied, as appropriate, to ensure data security.

The memory 16 is a data storage that can encompass any number and type of storage media suitable for storing computer-readable code or instructions, such as the aforementioned software program 30, as well as other data generally supporting the operation of the system 10. The memory 16 may also store one or more threshold 34 values, for use by an algorithm embodied in software program 30. One or more database(s) 28 are another form of storage media; they may be integrated with memory 16 or separate from it.

In various embodiments, aircraft-specific parameters and information for an aircraft may be stored in the memory 16 or in a database 28 and referenced by the program 30. Non-limiting examples of aircraft-specific information includes an aircraft weight and dimensions, performance capabilities, configuration options, and the like.

Flight parameter sensors and geospatial sensors 22 supply various types of data or measurements to the controller circuit 12 during an aircraft flight. In various embodiments, the geospatial sensors 22 supply, without limitation, one or more of: inertial reference system measurements providing a location, Flight Path Angle (FPA) measurements, airspeed data, groundspeed data (including groundspeed direction), vertical speed data, vertical acceleration data, altitude data, attitude data including pitch data and roll measurements, yaw data, heading information, sensed atmospheric conditions data (including wind speed and direction data), flight path data, flight track data, radar altitude data, and geometric altitude data.

With continued reference to FIG. 1, the display device 14 can include any number and type of image generating devices on which one or more avionic displays 32 may be produced. When the system 10 is utilized for a manned aircraft, the display device 14 may be affixed to the static structure of the Aircraft cockpit as, for example, a Head Down Display (HDD) or Head Up Display (HUD) unit. In various embodiments, the display device 14 may assume the form of a movable display device (e.g., a pilot-worn display device) or a portable display device, such as an Electronic Flight Bag (EFB), a laptop, or a tablet computer carried into the aircraft cockpit by a pilot.

At least one avionic display 32 is generated on the display device 14 during operation of the system 10; the term "avionic display" is synonymous with the term "aircraft-related display" and "cockpit display" and encompasses displays generated in textual, graphical, cartographical, and other formats. The system 10 can generate various types of lateral and vertical avionic displays 32 on which map views and symbology, text annunciations, and other graphics pertaining to flight planning are presented for a pilot to view. The display device 14 is configured to continuously render at least a lateral display showing the aircraft at its current location within the map data. The avionic display 32 generated and controlled by the system 10 can include graphical user interface (GUI) objects and alphanumerical input displays of the type commonly presented on the screens of multifunction control display units (MCDUs), as well as Control Display Units (CDUs) generally. Specifically, embodiments of the avionic displays 32 include one or more two-dimensional (2D) avionic displays, such as a horizontal (i.e., lateral) navigation display or vertical navigation display (i.e., vertical situation display VSD); and/or on one or more three dimensional (3D) avionic displays, such as a Primary Flight Display (PFD) or an exocentric 3D avionic display.

In various embodiments, a human-machine interface is implemented as an integration of a pilot input interface 18 and a display device 14. In various embodiments, the display device 14 is a touch screen display. In various embodiments, the human-machine interface also includes a separate pilot input interface 18 (such as a keyboard, cursor control device, voice input device, or the like), generally operationally coupled to the display device 14. Via various display and graphics systems processes, the controller circuit 12 may command and control a touch screen display device 14 to generate a variety of graphical user interface (GUI) objects or elements described herein, including, for example, buttons, sliders, and the like, which are used to prompt a user to interact with the human-machine interface to provide user input; and for the controller circuit 12 to activate respective functions and provide user feedback, responsive to received user input at the GUI element.

In various embodiments, the system 10 may also include a dedicated communications circuit 24 configured to provide a real-time bidirectional wired and/or wireless data exchange for the controller 12 to communicate with the external sources 50 (including, each of: traffic, air traffic control (ATC), satellite weather sources, ground stations, and the like). In various embodiments, the communications circuit 24 may include a public or private network implemented in accordance with Transmission Control Protocol/Internet Protocol architectures and/or other conventional protocol standards. Encryption and mutual authentication techniques may be applied, as appropriate, to ensure data security. In some embodiments, the communications circuit 24 is integrated within the controller circuit 12, and in other embodiments, the communications circuit 24 is external to the controller circuit 12. When the external source 50 is "traffic," the communications circuit 24 may incorporate software and/or hardware for communication protocols as needed for traffic collision avoidance (TCAS), automatic dependent surveillance broadcast (ADSB), and enhanced vision systems (EVS).

In certain embodiments of the system 10, the controller circuit 12 and the other components of the system 10 may be integrated within or cooperate with any number and type of systems commonly deployed onboard an aircraft including, for example, an FMS 21.

The disclosed algorithm is embodied in a hardware program or software program (e.g. program 30 in controller circuit 12) and configured to operate when the aircraft is in any phase of flight. The algorithm enables detection of potential physiological episodes (PE) based on sensed pilot respiration data in an aircraft.

In various embodiments, the provided controller circuit 12, and therefore its program 30 may incorporate the programming instructions for: receiving raw physiological data from at least one sensor of a biosensing garment of a pilot, the raw physiological data including raw respiration data; extracting individual breaths from the raw physiological data, each individual breath having an associated breath pattern and an association with a breath timeline; receiving aircraft environment data associated with an aircraft from at least one aircraft environment sensor, the aircraft environment data being associated with an aircraft environment timeline; aligning the individual breaths with the aircraft environment data in accordance with the breath timeline and the aircraft environment timeline; classifying each individual breath as one of a plurality of different breath types based on the associated breath pattern; determining whether the breath types associated with the individual breaths are associated with a physiological episode (PE) profile based at least in part on the aircraft environment data in accordance with the aligned breath and aircraft timelines; and issuing a PE alert based on the determination.

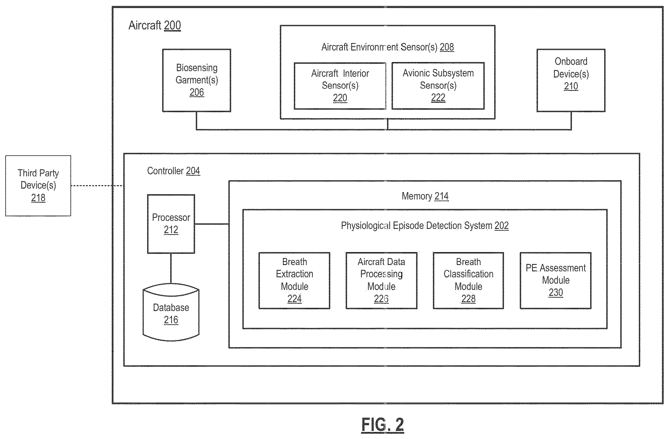

Referring to FIG. 2, a block diagram representation of an aircraft 200 including an embodiment of a physiological episode (PE) detection system 202 is shown. In an embodiment, the aircraft 200 includes a controller 204, a biosensing garment(s) 206, aircraft environment sensor(s) 208, and output device(s) 210. The controller 204 includes one or more processors 212 and a memory 214. In an embodiment, the controller 204 includes a database 216. The database 216 is configured to store potential physiological episode data of pilots detected by the (PE) detection system 202. The controller 204 is configured to be communicatively coupled to a third party device 218. Examples of the third party devices 218 include, but are not limited to, a pilot training display and an air traffic controller display. The memory 214 includes the (PE) detection system 202. In an embodiment, the aircraft 200 has a configuration similar to that of the platform 5. The aircraft 200 may include additional components that facilitate operation of the aircraft 200.

The pilot(s) on the aircraft 200 wears biosensing garment(s) 206. Each biosensing garment 206 includes one or more biosensors that are configured to sense raw physiological data including raw respiration data of a pilot. The PE detection system 202 is configured to receive the raw respiration data from the biosensing garment 206. In an embodiment, the biosensing garment 206 is configured to sense raw physiological data including raw respiration data and raw electrocardiogram data of the pilot. The PE detection system 202 is configured to receive the raw respiration data and the raw electrocardiogram data.

The aircraft environment sensors 208 are configured to sense aircraft environment data associated with the aircraft. The PE detection system 202 is configured to receive the aircraft environment data from at least one aircraft environment sensor 208. In an embodiment, the aircraft environment sensors 208 include one or more aircraft interior sensors 220. In an embodiment, the aircraft environment sensors 208 include one or more avionic subsystem sensors 222. In an embodiment, the aircraft environment sensors 208 include one or more aircraft interior sensors 220 and one or more avionic subsystem sensors 222.

The aircraft interior sensors 220 include at least one of life support system sensors, oxygen level sensors, nitrogen level sensors, and cabin pressure sensors. The PE detection system 202 is configured to receive aircraft environment data associated with the aircraft 200 from at least one of the life support system sensors, the oxygen level sensors, the nitrogen level sensors, and the cabin pressure sensors. In an embodiment, the aircraft environment data includes at least one of life support system data, oxygen level data, nitrogen level data, and cabin pressure data. In alternative embodiments, the aircraft interior sensors 220 may include additional types of aircraft interior sensors 220 that provide aircraft environment data to the PE detection system 202.

The avionic subsystem sensors 222 include at least one type of ownship data source 22. In an embodiment, the avionic subsystem sensors 222 include aircraft motion data sensors. The PE detection system 202 is configured to receive aircraft motion data from the aircraft motion sensors. The PE detection system 202 is configured to generate aircraft g-force data based on the aircraft motion data. In an embodiment, the avionic subsystem sensors 222 include aircraft g-force data sensors. The PE detection system 202 is configured to receive aircraft g-force data from the aircraft g-force data sensors.

The aircraft 200 includes one or more output devices 210. The output device 210 is configured to generate a PE alert. In an embodiment, when the PE detection system 202 detects a potential pilot PE, the PE detection system 202 is configured to issue a PE alert via an output device 210. Examples of PE alerts include, but are not limited to, a pilot PE alert, a cockpit haptic PE alert, a cockpit audio PE alert, and a cockpit display PE alert. In an embodiment, when the PE detection system 202 detects a potential pilot PE episode, the PE detection system 202 is configured to issue a PE alert to a third party device 218. Examples of the third party devices 218 include, but are not limited to, a pilot training display and an air traffic controller display.

The controller 204 includes a processor 212 and a memory 214. The memory 214 includes an embodiment of the PE detection system 202. In an embodiment, the PE detection system 202 includes a breath extraction module 224, an aircraft data processing module 226, a breath classification module 228, and a PE assessment module 230. The PE detection system 202 may include additional components that facilitate operation of the PE detection system 202.

The breath extraction module 224 is configured to receive raw physiological data from at least one sensor of a biosensing garment 206 of a pilot. In an embodiment, the raw physiological data is raw respiration data of the pilot. In an embodiment, the raw physiological data includes raw respiration data and electrocardiogram data of the pilot. In an embodiment, the breath data extraction module 224 is configured to process the raw physiological data by applying a noise filter to the raw physiological data.

The breath data extraction module 224 is configured to extract individual breaths from the raw physiological data. Each individual breath has a breath pattern. The individual breaths are associated with a breath timeline. The breath timeline is based on the time at which the raw respiration data associated with the individual breaths was received from the biosensing garment 206 at the PE detection system 202.

The aircraft data processing module 226 is configured to receive aircraft environment data associated with the aircraft from at least one aircraft environment sensor 208. In an embodiment, the aircraft data processing module 226 is configured to receive aircraft environment data from at least one aircraft interior sensor 220. In an embodiment, the aircraft data processing module 226 is configured to receive aircraft environment data from at least one of life support system sensors, oxygen level sensors, nitrogen level sensors, and cabin pressure sensors.

In an embodiment, the aircraft data processing module 226 is configured to receive aircraft environment data from at least one avionic subsystem sensor 222. The avionic subsystem sensors 234 include one or more different ownship data sources 22. In an embodiment, the aircraft data processing module 226 is configured to receive aircraft motion data from an avionic subsystem 222. The aircraft data processing module 226 is configured to generate aircraft g-force data based on the aircraft motion data. In an embodiment, the aircraft data processing module 226 is configured to receive aircraft g-force data from an avionic subsystem 222. In an embodiment, the aircraft data processing module 226 is configured to receive aircraft environment data from at least one aircraft interior sensor 220 and at least one avionic subsystem sensor 222.

**In** an embodiment, the aircraft data processing module 226 is configured to apply a noise filter to the aircraft environment data. In an embodiment, the aircraft data processing module 226 is configured to apply a smoothing algorithm to the aircraft environment data. In an embodiment, the aircraft data processing module 226 is configured to apply a noise filter and a smoothing algorithm to the aircraft environment data. The aircraft environment data is associated with an environment data timeline. The environment data timeline is based on the time at which the aircraft environment data was received at the PE detection system 202.

The breath classification module 228 is configured to receive the individual breaths from the breath extraction module 224. Each individual breath has a breath pattern. The breath classification module 228 is configured to classify each individual breath as one of a plurality of different breath types based on the associated breath pattern. Examples of the different types of breaths include, but are not limited to shallow breathing, hyperventilation breathing, coughing breathing, talking breathing, deep breathing, normal breathing, apnea, big breath and anti-G straining maneuver (AGSM).

In an embodiment, the breath classification module 228 is configured to employ a trained breath classification neural network to classify each individual breath as one of the plurality of different breath types based on the associated breath pattern. The breath classification module 228 is configured to generate at least one breath feature for a breath pattern of an individual breath. The breath classification module 228 is configured to employ the trained breath classification neural network to classify the individual breath as one of the plurality of different individual breaths based on the at least one breath feature.

In an embodiment, the breath classification module 228 is configured to employ a decision tree to classify each individual breath as one of the plurality of different breath types. In an embodiment, the breath classification module 228 is configured to employ a regression model to classify each of the individual breaths as one of the plurality of different breath types. In an embodiment, the breath classification module 228 is configured to classify each individual breath as one of the plurality of different breath types where each different breath type is a state. The breath classification module 228 is configured to classify each individual breath as one of the plurality of different breath types based on the application of state detection with confidence thresholds.

The PE assessment module 230 is configured to receive the breath types associated with each of the individual breaths and the associated breath timeline. The PE assessment module 230 is configured to receive the aircraft environment data and the associated environment data timeline. The PE assessment module 230 is configured to align the breath types of the extracted individual breaths with the aircraft environment data in accordance with the breath timeline with the environment data timeline. Upon alignment, the time at which the raw respiration data associated with the breath type of the individual breath was received at the PE detection system 202 matches the time at which the environment data was received at the PE detection system 202.

The PE assessment module 230 includes a plurality of PE profiles. Each PE profile represents an association between a breath type and aircraft environment data that may be indicative of a potential PE episode of a pilot. The PE assessment module 230 is configured to determine whether a received breath type of an individual breath associated with aircraft environment data in accordance with the aligned breath timeline and environment data timeline matches one of the plurality of PE profiles.

In an embodiment, the raw physiological data received from the biosensing garment 206 includes electrocardiogram data. In an embodiment, each PE profile represents an association between a breath type, a heart rate, and aircraft environment data that may be indicative of a potential PE episode of a pilot. The PE assessment module 230 is configured to determine whether a received breath type of an individual breath associated with a heart rate, and the aircraft environment data in accordance with the aligned breath timeline and environment data timeline matches one of the plurality of PE profiles.

In an embodiment, the raw physiological data received from the biosensing garment 206 includes electrocardiogram data. In an embodiment, each PE profile represents an association between a breath type, a heart rate variability, and aircraft environment data that may be indicative of a potential PE episode of a pilot. The PE assessment module 230 is configured to determine whether a received breath type of an individual breath associated with a heart rate variability, and aircraft environment data in accordance with the aligned breath timeline and environment data timeline matches one of the plurality of PE profiles.

If the PE assessment module 230 determines that there is a match with one of the plurality of PE profiles, the PE assessment module 230 is configured to issue a PE alert. Examples of PE alerts include, but are not limited to, a cockpit haptic PE alert, a cockpit audio PE alert, and a cockpit display PE alert. In an embodiment, the PE alert is pilot PE alert indicating that a potential PE episode may occur. In an embodiment, the PE alert indicates that the pilot may be potentially experiencing a PE.

In an embodiment, the PE alert is an instruction to a pilot to alter his/her breath type to a different breath type to avoid the potential PE episode. In an embodiment, the PE assessment module 230 is configured to issue the PE alert to a third party device 218. Examples of the third party devices 218 include, but are not limited to, a pilot training display and an air traffic controller display.

In an embodiment, if the PE assessment module 230 determines that there is a match with one of the plurality PE profiles, the PE assessment module 230 is configured to store one or more of the breath types associated with the extracted individual breaths, the breath timeline, the aircraft environment data, and the environment data timeline used to make the determination, and the matched PE profile in the database 216. In an embodiment, if the aircraft 200 is involved in a crash or an accident, the stored data can be used to assess whether there is a correlation between the crash/accident and the occurrence of a pilot PE episode.

In an example, the PE assessment module 230 may receive aircraft environment data that may be aircraft g-force data associated with an aircraft environment timeline. The aircraft g-force data may have a value where a pilot typically engages in AGSM. The PE assessment module 230 may receive a breath type associated with the extracted individual breath and a breath timeline indicating that the pilot is not engaging in AGSM. The aircraft environment data is aligned with the breath type of the associated extracted individual breath in accordance with the aircraft environment timeline and the breath timeline. A PE profile may indicate that aircraft g-force data combined with breath type indicating that the pilot is not engaging in AGSM may lead to a potential pilot PE. The PE assessment module 230 may determine that the received breath type of the extracted individual breath and aircraft environment data in accordance with the aligned breath timeline and aircraft environment timeline matches the described PE profile and issue an AGSM PE alert on a cockpit display and/or a pilot training display

In an embodiment, the PE assessment module 230 is configured to determine whether the matched PE profile corresponds to a pilot incapacitating PE episode. If the PE assessment module 230 determines that the matched PE profile corresponds to a pilot incapacitating PE episode, the PE assessment module 230 is configured to automatically implement an aircraft control action. Examples of aircraft control actions include, but are not limited to, a life support system action and an aircraft control action. An example of a life support system action is adjusting one or more of the oxygen levels, the nitrogen levels, and the cabin pressure in the aircraft 200. An example of an aircraft control action is placing the aircraft 200 in autopilot.

In an embodiment, the PE detection system 202 is configured to issue the PE alert in accordance with some communication criteria. The communication criteria includes at least one of a pilot behavior associated with the breath types, an urgency criterion, and an aircraft environmental parameter associated with the breath types.

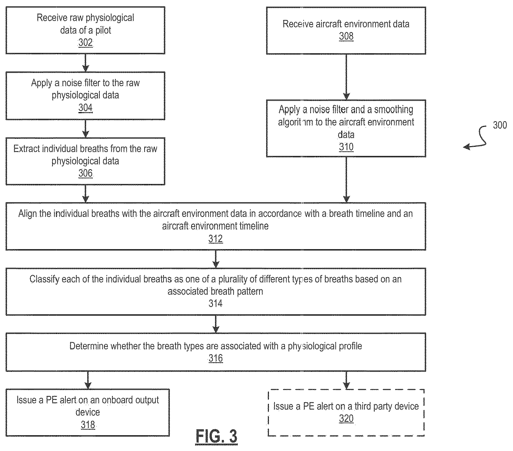

Referring to FIG. 3, a flowchart representation of an exemplary embodiment of a method 300 (not part of the invention) of detecting potential physiological episodes based on sensed pilot respiration data is shown. The method 300 (not part of the invention) is implemented by an embodiment of the PE detection system 202.

At 302, the PE detection system 202 receives raw physiological data from a biosensing garment 206 of a pilot. The raw physiological data includes raw respiration data of the pilot. At 304, the PE detection system 202 applies a noise filter to the raw physiological data. At 306, the PE detection system 202 extracts individual breaths from the raw physiological data. Each individual breath has an associated breath pattern. Each individual breath is associated with a breath timeline indicating a time at which the raw physiological data associated with the individual breath was received at the PE detection system 202.

At 308, the PE detection system 202 receives aircraft environment data from at least one aircraft environment sensor 208. Examples of aircraft environment sensors 208 include aircraft interior sensors 220 and avionic system sensors 222. The aircraft environment data is associated with an aircraft environment timeline indicating a time at which the aircraft environment data was received at the PE detection system 202. At 310, the PE detection system 202 applies a noise filter and a smoothing algorithm to the aircraft environment data. In an embodiment, the raw physiological data of the pilot and the aircraft environment data are received at the PE detection system 202 at approximately the same time.

At 312, the PE detection system 202 aligns the individual breaths with the aircraft environment data in accordance with the breath timeline and the aircraft environment timeline. At 314, the PE detection system 202 classifies each individual breath as one of a plurality of breath types based on the associated breath pattern. Examples of the plurality of breath types include, but are not limited to, shallow breathing, hyperventilation breathing, coughing breathing, talking breathing, deep breathing, normal breathing, apnea, big breath, and AGSM. The PE detection system 202 uses one of a trained breath classification neural network, a decision tree, and a regression model to classify the individual breaths.

At 316, the PE detection system 202 determines whether the breath types associated with the individual breaths are associated with a PE profile based on the aircraft environment data in accordance with the aligned breath and aircraft environment timelines. At 318, the PE detection system 202 issues a PE alert on an onboard output device 210 based on the determination. Examples of PE alerts include, but are not limited to, a cockpit haptic PE alert, a cockpit audio PE alert, and a cockpit display PE alert. The PE alert may be an AGSM alert. The PE alert may be a potential PE alert. The PE alert may be a pilot incapacitated alert.

Optionally at 320, the PE detection system 202 issues a PE alert on a third party device. Examples of third party devices include, but are not limited to, a pilot training display and an air traffic controller display.

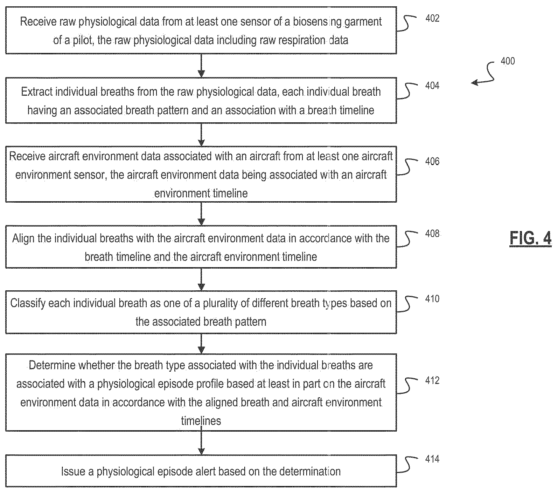

Referring to FIG. 4, a flowchart representation of an exemplary embodiment of a method 400 (not part of the invention) of detecting potential physiological episodes based on sensed pilot respiration data is shown. The method 400 (not part of the invention) is implemented by an embodiment of the PE detection system 202. At 402, raw physiological data is received from at least one sensor of a biosensing garment 206 of a pilot. The raw physiological data includes raw respiration data. At 404, individual breaths are extracted from the raw physiological data. Each individual breath has an associated breath pattern and an association with a breath timeline. At 406, aircraft environment data associated with an aircraft 200 is received from at least one aircraft environment sensor 208. The aircraft environment data is associated with an aircraft environment timeline. At 408, the individual breaths are aligned with the aircraft environment data in accordance with the breath timeline and the aircraft environment timeline. At 410, each individual breath is classified as one of a plurality of different breath types based on the associated breath pattern. At 412, a determination is made regarding whether the breath types associated with the individual breaths are associated with a physiological episode (PE) profile based at least in part on the aircraft environment data in accordance with the aligned breath and aircraft timelines. At 414, a PE alert is issued based on the determination.

Detecting physiological states of a pilot and a potential onset of physiological episodes may enable the implementation of appropriate action by the pilot or by automation and avoid potential adverse aircraft events. Certain breathing states (also referred to as breathing types) associated with pilot breathing patterns are often indicative of changes in physiological states of a pilot and the potential for the onset of a physiological episode. High physical and mental demands can induce changes in normal breathing patterns of pilots. The potential onset of a physiological episode can be detected through an analysis of pilot respiration data in the context of an aircraft environment enabling the implementation of action in a timely manner to ensure the safety of the pilot and the aircraft.

The use of biosensing apparel to capture raw pilot respiration data, extract individual breaths, and provide classification of individual breaths may provide insight into pilot lung functioning. The classification of the extracted breaths focuses on a core set of breathing behaviors of interest in the context of the aircraft environment related to the potential onset of physiological episodes. The classification is used to provide actionable information to the pilot or the aircraft systems.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations.

The various illustrative logical blocks, modules, and circuits described in connection with the embodiments disclosed herein may be implemented or performed with a general-purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC.

Techniques and technologies may be described herein in terms of functional and/or logical block components, and with reference to symbolic representations of operations, processing tasks, and functions that may be performed by various computing components or devices. Such operations, tasks, and functions are sometimes referred to as being computer-executed, computerized, software-implemented, or computer-implemented. In practice, one or more processor devices can carry out the described operations, tasks, and functions by manipulating electrical signals representing data bits at memory locations in the system memory, as well as other processing of signals. The memory locations where data bits are maintained are physical locations that have particular electrical, magnetic, optical, or organic properties corresponding to the data bits. It should be appreciated that the various block components shown in the figures may be realized by any number of hardware, software, and/or firmware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices.

When implemented in software or firmware, various elements of the systems described herein are essentially the code segments or instructions that perform the various tasks. The program or code segments can be stored in a processor-readable medium or transmitted by a computer data signal embodied in a carrier wave over a transmission medium or communication path. The "computer-readable medium", "processor-readable medium", or "machine-readable medium" may include any medium that can store or transfer information. Examples of the processor-readable medium include an electronic circuit, a semiconductor memory device, a ROM, a flash memory, an erasable ROM (EROM), a floppy diskette, a CD-ROM, an optical disk, a hard disk, a fiber optic medium, a radio frequency (RF) link, or the like. The computer data signal may include any signal that can propagate over a transmission medium such as electronic network channels, optical fibers, air, electromagnetic paths, or RF links. The code segments may be downloaded via computer networks such as the Internet, an intranet, a LAN, or the like.

Some of the functional units described in this specification have been referred to as "modules" in order to more particularly emphasize their implementation independence. For example, functionality referred to herein as a module may be implemented wholly, or partially, as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices, or the like. Modules may also be implemented in software for execution by various types of processors. An identified module of executable code may, for instance, comprise one or more physical or logical modules of computer instructions that may, for instance, be organized as an object, procedure, or function. Nevertheless, the executables of an identified module need not be physically located together but may comprise disparate instructions stored in different locations that, when joined logically together, comprise the module and achieve the stated purpose for the module. Indeed, a module of executable code may be a single instruction, or many instructions, and may even be distributed over several different code segments, among different programs, and across several memory devices. Similarly, operational data may be embodied in any suitable form and organized within any suitable type of data structure. The operational data may be collected as a single data set or may be distributed over different locations including over different storage devices, and may exist, at least partially, merely as electronic signals on a system or network.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order which is encompassed by the appended claims.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. The invention is solely defined by the appended claims.

## Claims

1. A system comprising:
a processor (212); and
a memory (214), the memory comprising instructions that upon execution by the processor, cause the processor to:
receive (302, 402) raw physiological data from at least one sensor of a biosensing garment of a pilot, the raw physiological data comprising raw respiration data;
extract (306, 404) individual breaths from the raw physiological data, each individual breath having an associated breath pattern and an association with a breath timeline;
receive (308, 406) aircraft environment data associated with an aircraft from at least one aircraft environment sensor, the aircraft environment data being associated with an aircraft environment timeline;
align (312, 408) the individual breaths with the aircraft environment data in accordance with the breath timeline and the aircraft environment timeline;
classify (314, 410) each individual breath as one of a plurality of different breath types based on the associated breath pattern;
determine (316, 412) whether the breath types associated with the individual breaths are associated with a physiological episode, PE, profile based at least in part on the aircraft environment data in accordance with the aligned breath and aircraft timelines; and
issue (318, 320, 414) a PE alert based on the determination.

2. The system of claim 1, wherein the memory further comprises instructions that upon execution by the processor, cause the processor to apply (304) a first noise filter to the raw respiration data prior to extraction of the individual breaths from the raw physiological data.

3. The system of claim 1, wherein the memory further comprises instructions that upon execution by the processor, cause the processor to receive the aircraft environment data from at least one of an aircraft motion sensor, an aircraft g-force sensor, a life support system sensor, an oxygen level sensor, a nitrogen level sensor, and a cabin pressure sensor.

4. The system of claim 1, wherein the memory further comprises instructions that upon execution by the processor, cause the processor to apply (310) a smoothing algorithm to the aircraft environmental data.

5. The system of claim 1, wherein the memory further comprises instructions that upon execution by the processor, cause the processor to apply (310) a second noise filter to the aircraft environmental data.

6. The system of claim 1, wherein the memory further comprises instructions that upon execution by the processor, cause the processor to:
generate at least one breath feature associated with a first breath pattern of a first individual breath; and
employ a trained breath classification neural network to classify the first individual breath as one of the plurality of different breath types based on the at least one breath feature.

7. The system of claim 1, wherein the memory further comprises instructions that upon execution by the processor, cause the processor to classify a first individual breath as one of a plurality of different breath types based on an associated first breath pattern using at least one of a decision tree and a regression model.

8. The system of claim 1, wherein the memory further comprises instructions that upon execution by the processor, cause the processor to:
make a first determination regarding whether the aircraft environment data comprises at least one of aircraft motion data associated with generation of aircraft g-forces and aircraft g-force data;
make a second determination regarding whether the breath types associated with the individual breaths comprise an anti-G straining maneuver, AGSM; and
issue the PE alert based on the first and second determination, the PE alert comprising an AGSM alert.

9. The system of claim 1, wherein the memory further comprises instructions that upon execution by the processor, cause the processor to classify each individual breath as one of the plurality of different breath types based on the associated breath pattern, the plurality of different breath types comprising shallow breathing, hyperventilation breathing, coughing breathing, talking breathing, deep breathing, normal breathing, apnea, big breath, and AGSM.

10. The system of claim 1, wherein the memory further comprises instructions that upon execution by the processor, cause the processor to:
receive the raw physiological data from at least one sensor of the biosensing garment of the pilot, the raw physiological data comprising electrocardiogram data;
calculate at least one of heart rate and heart rate variability based on the electrocardiogram data; and
determine whether the breath types associated with the individual breaths are associated with the PE profile based in part on at least one of the heart rate and the heart rate variability.

11. The system of claim 1, wherein the memory further comprises instructions that upon execution by the processor, cause the processor to store the determination regarding whether the breath types associated with the individual breaths are associated with the PE profile in a database to enable an assessment of whether there is a correlation between the PE profile and an aircraft event.

12. The system of claim 1, wherein the PE alert comprises a potential PE episode alert.

13. The system of claim 1, wherein the memory further comprises instructions that upon execution by the processor, cause the processor to:
determine whether the breath types associated with the individual breaths is associated with the PE profile, the PE profile being associated with a pilot incapacitating PE; and
automatically implement aircraft action comprising at least one of a life support system action and an aircraft control action based on the determination.

14. The system of claim 1, wherein the memory further comprises instructions that upon execution by the processor, cause the processor to issue the PE alert in accordance with some communication criteria, the communication criteria comprising at least one of a pilot behavior associated with the breath types, an urgency criterion, and an aircraft environmental parameter associated with the breath types.

## Patentansprüche

1. System, umfassend:
einen Prozessor (212); und
einen Speicher (214), wobei der Speicher Anweisungen umfasst, die bei Ausführung durch den Prozessor bewirken, dass der Prozessor:
physiologische Rohdaten von mindestens einem Sensor eines Biosensorik-Kleidungsstücks eines Piloten empfängt (302, 402), wobei die physiologischen Rohdaten Atmungsrohdaten umfassen;
einzelne Atemzüge aus den physiologischen Rohdaten extrahiert (306, 404), wobei jeder einzelne Atemzug ein verbundenes Atemzugmuster und eine Verbindung zu einer Atemzugzeitachse aufweist;
Flugzeugumgebungsdaten, die mit einem Flugzeug verbunden sind, von mindestens einem Flugzeugumgebungssensor empfängt (308, 406), wobei die Flugzeugumgebungsdaten mit einer Flugzeugumgebungszeitachse verbunden sind;
die einzelnen Atemzüge an den Flugzeugumgebungsdaten in Übereinstimmung mit der Atemzeitachse und der Flugzeugumgebungszeitachse ausrichtet (312, 408);
jeden einzelnen Atemzug basierend auf dem verbundenen Atemmuster als einen aus einer Vielzahl verschiedener Atemzugtypen klassifiziert (314, 410);
bestimmt (316, 412), ob die mit den einzelnen Atemzügen verbundenen Atemzugtypen mit einem physiologisches-Ereignis-, PE, -Profil verbunden sind, welches zumindest teilweise auf den Flugzeugumgebungsdaten in Übereinstimmung mit den ausgerichteten Atemzug- und Flugzeugzeitachsen basiert; und
einen PE-Alarm basierend auf der Bestimmung ausgibt (318, 320, 414).

2. System nach Anspruch 1, wobei der Speicher ferner Anweisungen umfasst, die bei Ausführung durch den Prozessor bewirken, dass der Prozessor einen ersten Rauschfilter auf die Atmungsrohdaten anwendet (304), bevor die einzelnen Atemzüge aus den physiologischen Rohdaten extrahiert werden.

3. System nach Anspruch 1, wobei der Speicher ferner Anweisungen umfasst, die bei Ausführung durch den Prozessor bewirken, dass der Prozessor die Flugzeugumgebungsdaten von mindestens einem der folgenden empfängt: einem Flugzeugbewegungssensor, einem Flugzeug-G-Kraft-Sensor, einem Lebenserhaltungssystemsensor, einem Sauerstoffgehaltsensor, einem Stickstoffgehaltsensor und einem Kabinendrucksensor.

4. System nach Anspruch **1,** wobei der Speicher ferner Anweisungen umfasst, die bei Ausführung durch den Prozessor bewirken, dass der Prozessor einen Glättungsalgorithmus auf die Flugzeugumgebungsdaten anwendet (310).

5. System nach Anspruch **1,** wobei der Speicher ferner Anweisungen umfasst, die bei Ausführung durch den Prozessor bewirken, dass der Prozessor einen zweiten Rauschfilter auf die Flugzeugumgebungsdaten anwendet (310).

6. System nach Anspruch **1,** wobei der Speicher ferner Anweisungen umfasst, die bei Ausführung durch den Prozessor bewirken, dass der Prozessor:
mindestens ein Atemzugmerkmal erzeugt, das mit einem ersten Atemzugmuster eines ersten individuellen Atemzugs verbunden ist; und
ein trainiertes neuronales Netzwerk zur Atemzugklassifizierung einsetzt, um den ersten individuellen Atemzug basierend auf dem mindestens einen Atemzugmerkmal als einen aus der Vielzahl verschiedener Atemzugtypen zu klassifizieren.

7. System nach Anspruch 1, wobei der Speicher ferner Anweisungen umfasst, die bei Ausführung durch den Prozessor bewirken, dass der Prozessor einen ersten individuellen Atemzug als einen aus einer Vielzahl verschiedener Atemzugtypen klassifiziert, basierend auf einem verbundenen ersten Atemzugmuster mittels mindestens eines der Folgenden: einem Entscheidungsbaum und einem Regressionsmodell.

8. System nach Anspruch 1, wobei der Speicher ferner Anweisungen umfasst, die bei Ausführung durch den Prozessor bewirken, dass der Prozessor:
eine erste Bestimmung in Bezug darauf vornimmt, ob die Flugzeugumgebungsdaten mindestens eines der folgenden umfassen: Flugzeugbewegungsdaten, die mit der Erzeugung von Flugzeug-G-Kräften verbunden sind, und Flugzeug-G-Kraftdaten;
eine zweite Bestimmung in Bezug darauf vornimmt, ob die mit den einzelnen Atemzügen verbundenen Atemzugtypen ein Anti-G-Belastungsmanöver, AGSM, umfassen; und
den PE-Alarm basierend auf der ersten und zweiten Bestimmung ausgibt, wobei der PE-Alarm einen AGSM-Alarm umfasst.

9. System nach Anspruch 1, wobei der Speicher ferner Anweisungen umfasst, die bei Ausführung durch den Prozessor bewirken, dass der Prozessor jeden einzelnen Atemzug basierend auf dem verbundenen Atemzugmuster als einen aus der Vielzahl verschiedener Atemzugtypen klassifiziert, wobei die Vielzahl verschiedener Atemzugtypen flaches Atmen, Hyperventilationsatmen, Hustenatmen, Sprechatmen, tiefes Atmen, normales Atmen, Apnoe, einen tiefen Atemzug und AGSM umfasst.

10. System nach Anspruch 1, wobei der Speicher ferner Anweisungen umfasst, die bei Ausführung durch den Prozessor bewirken, dass der Prozessor:
die physiologischen Rohdaten von mindestens einem Sensor des Biosensorik-Kleidungsstücks des Piloten empfängt, wobei die physiologischen Rohdaten Elektrokardiogrammdaten umfassen;
basierend auf den Elektrokardiogrammdaten mindestens eines der folgenden berechnet: Herzfrequenz und Herzfrequenzvariabilität; und
bestimmt, ob die mit den einzelnen Atemzügen verbundenen Atemzugtypen mit dem PE-Profil verbunden sind, basierend teilweise auf mindestens einem der folgenden: Herzfrequenz und Herzfrequenzvariabilität.

11. System nach Anspruch 1, wobei der Speicher ferner Anweisungen umfasst, die bei Ausführung durch den Prozessor bewirken, dass der Prozessor die Bestimmung in Bezug darauf speichert, ob die mit den einzelnen Atemzügen verbundenen Atemzugtypen mit dem PE-Profil in einer Datenbank verbunden sind, um eine Bewertung zu ermöglichen, ob es eine Korrelation zwischen dem PE-Profil und einem Flugzeugereignis gibt.

12. System nach Anspruch 1, wobei der PE-Alarm einen möglichen PE-Ereignisalarm umfasst.

13. System nach Anspruch 1, wobei der Speicher ferner Anweisungen umfasst, die bei Ausführung durch den Prozessor bewirken, dass der Prozessor:
bestimmt, ob die mit den einzelnen Atemzügen verbundenen Atemzugtypen mit dem PE-Profil verbunden sind, wobei das PE-Profil mit einem Piloten außer Gefecht setzenden PE verbunden ist; und
automatisch eine Flugzeugaktion implementiert, die basierend auf der Bestimmung mindestens eines der folgenden umfasst: eine Lebenserhaltungssystemaktion und eine Flugzeugsteuerungsaktion.

14. System nach Anspruch 1, wobei der Speicher ferner Anweisungen umfasst, die bei Ausführung durch den Prozessor bewirken, dass der Prozessor den PE-Alarm in Übereinstimmung mit einigen Kommunikationskriterien ausgibt, wobei die Kommunikationskriterien mindestens eines der folgenden umfassen: ein mit den Atemzugtypen verbundenes Pilotenverhalten, ein Dringlichkeitskriterium und einen mit den Atemzugtypen verbundenen Flugzeugumgebungsparameter.

## Revendications

1. Système comprenant :
un processeur (212) ; et
une mémoire (214), la mémoire comprenant des instructions qui, après exécution par le processeur, amènent le processeur à :
recevoir (302, 402) des données physiologiques brutes provenant d'au moins un capteur d'un vêtement de biodétection d'un pilote, les données physiologiques brutes comprenant des données respiratoires brutes ;
extraire (306, 404) des respirations individuelles à partir des données physiologiques brutes, chaque respiration individuelle ayant un motif de respiration associé et une association avec une chronologie des respirations ;
recevoir (308, 406) des données d'environnement de l'aéronef associées à un aéronef provenant d'au moins un capteur d'environnement de l'aéronef, les données d'environnement de l'aéronef étant associées à une chronologie d'environnement de l'aéronef ;
aligner (312, 408) les respirations individuelles avec les données d'environnement de l'aéronef conformément à la chronologie des respirations et à la chronologie d'environnement de l'aéronef ;
classifier (314, 410) chaque respiration individuelle comme étant l'un d'une pluralité de différents types de respiration sur la base du motif de respiration associé ;
déterminer (316, 412) si les types de respiration associés aux respirations individuelles sont associés à un profil d'épisode physiologique, PE, sur la base, au moins en partie, des données d'environnement de l'aéronef conformément aux chronologies des respirations et d'aéronef alignées ; et
émettre (318, 320, 414) une alerte PE sur la base de la détermination.

2. Système selon la revendication 1, dans lequel la mémoire comprend en outre des instructions qui, après exécution par le processeur, amènent le processeur à appliquer (304) un premier filtre de bruit aux données respiratoires brutes avant l'extraction des respirations individuelles à partir des données physiologiques brutes.

3. Système selon la revendication 1, dans lequel la mémoire comprend en outre des instructions qui, après exécution par le processeur, amènent le processeur à recevoir les données d'environnement de l'aéronef provenant d'au moins l'un parmi un capteur de mouvement de l'aéronef, un capteur de forces G de l'aéronef, un capteur de système de survie, un capteur de niveau d'oxygène, un capteur de niveau d'azote et un capteur de pression de cabine.

4. Système selon la revendication 1, dans lequel la mémoire comprend en outre des instructions qui, après exécution par le processeur, amènent le processeur à appliquer (310) un algorithme de lissage aux données environnementales de l'aéronef.

5. Système selon la revendication 1, dans lequel la mémoire comprend en outre des instructions qui, après exécution par le processeur, amènent le processeur à appliquer (310) un deuxième filtre de bruit aux données environnementales de l'aéronef.

6. Système selon la revendication 1, dans lequel la mémoire comprend en outre des instructions qui, après exécution par le processeur, amènent le processeur à :
générer au moins une caractéristique de respiration associée à un premier motif de respiration d'une première respiration individuelle ; et
utiliser un réseau neuronal de classification des respirations entraîné pour classifier la première respiration individuelle comme étant l'un de la pluralité de différents types de respiration sur la base de ladite au moins une caractéristique de respiration.

7. Système selon la revendication 1, dans lequel la mémoire comprend en outre des instructions qui, après exécution par le processeur, amènent le processeur à classifier une première respiration individuelle comme étant l'un d'une pluralité de différents types de respiration sur la base d'un premier motif de respiration associé en utilisant au moins l'un parmi un arbre de décision et un modèle de régression.

8. Système selon la revendication 1, dans lequel la mémoire comprend en outre des instructions qui, après exécution par le processeur, amènent le processeur à :
effectuer une première détermination visant à établir si les données d'environnement de l'aéronef comprennent au moins l'une parmi des données de mouvement de l'aéronef associées à la génération de forces G de l'aéronef et des données de forces G de l'aéronef ;
effectuer une deuxième détermination visant à établir si les types de respiration associés aux respirations individuelles comprennent une manœuvre anti-G de poussée, AGSM ; et
émettre l'alerte PE sur la base de la première et de la deuxième détermination, l'alerte PE comprenant une alerte AGSM.

9. Système selon la revendication 1, dans lequel la mémoire comprend en outre des instructions qui, après exécution par le processeur, amènent le processeur à classifier chaque respiration individuelle comme étant l'un de la pluralité de différents types de respiration sur la base du motif de respiration associé, la pluralité de différents types de respiration comprenant une respiration superficielle, une respiration d'hyperventilation, une respiration de toux, une respiration de parole, une respiration profonde, une respiration normale, une apnée, une grande respiration et une AGSM.

10. Système selon la revendication 1, dans lequel la mémoire comprend en outre des instructions qui, après exécution par le processeur, amènent le processeur à :
recevoir les données physiologiques brutes provenant d'au moins un capteur du vêtement de biodétection du pilote, les données physiologiques brutes comprenant des données d'électrocardiogramme ;
calculer au moins l'une parmi une fréquence cardiaque et une variabilité de la fréquence cardiaque sur la base des données d'électrocardiogramme ; et
déterminer si les types de respiration associés aux respirations individuelles sont associés au profil PE en partie sur la base d'au moins l'une parmi la fréquence cardiaque et la variabilité de la fréquence cardiaque.

11. Système selon la revendication 1, dans lequel la mémoire comprend en outre des instructions qui, après exécution par le processeur, amènent le processeur à stocker la détermination visant à établir si les types de respiration associés aux respirations individuelles sont associés au profil PE dans une base de données afin de permettre une évaluation visant à établir s'il existe une corrélation entre le profil PE et un événement d'aéronef.

12. Système selon la revendication 1, dans lequel l'alerte PE comprend une alerte d'épisode PE potentiel.

13. Système selon la revendication 1, dans lequel la mémoire comprend en outre des instructions qui, après exécution par le processeur, amènent le processeur à :
déterminer si les types de respiration associés aux respirations individuelles sont associés au profil PE, le profil PE étant associé à un PE incapacitant pour le pilote ; et
mettre automatiquement en œuvre une action de l'aéronef comprenant au moins l'une parmi une action du système de survie et une action de commande de l'aéronef sur la base de la détermination.

14. Système selon la revendication 1, dans lequel la mémoire comprend en outre des instructions qui, après exécution par le processeur, amènent le processeur à émettre l'alerte PE conformément à certains critères de communication, les critères de communication comprenant au moins l'un parmi un comportement du pilote associé aux types de respiration, un critère d'urgence et un paramètre environnemental de l'aéronef associé aux types de respiration.
